# EUROPÄISCHE PATENTSCHRIFT

(11) **EP 2 313 023 B1**
(45) Veröffentlichungstag und Bekanntmachung des Hinweises auf die Patenterteilung: **12.12.2018**
(21) Anmeldenummer: 09800044.1
(22) Anmeldetag: 21.07.2009
(51) Int. Cl.: A61C 13/00, A61C 13/08

(54) **FORMKÖRPER AUS FORMSTABILISIERTEM MATERIAL UND VERFAHREN ZU SEINER HERSTELLUNG**
MOLDED MEMBER MADE OF FORM-STABILIZED MATERIAL AND METHOD FOR THE MANUFACTURE THEREOF
CORPS MOULÉ FABRIQUÉ À PARTIR DE MATÉRIAU INDÉFORMABLE ET SON PROCÉDÉ DE FABRICATION

(30) Priorität: 21.07.2008 EP 08160834; 28.10.2008 WO PCT/EP2008/064602
(43) Veröffentlichungstag der Anmeldung: 27.04.2011
(73) Patentinhaber: Vita Zahnfabrik H. Rauter GmbH & Co. KG, 79713 Bad Säckingen (DE)
(72) Erfinder: THIEL, Norbert, 79713 Bad Säckingen (DE); ALBARSKI, Olivia, 5000 Aarau (CH); DORN, Michael, 72393 Burladingen (DE); AECHTNER, Stefan, 79713 Bad Säckingen (DE); BIBUS, Joachim, 79713 Bad Säckingen (DE); SCHMID, Andreas, 79713 Bad Säckingen (DE)
(74) Vertreter: dompatent von Kreisler Selting Werner - Partnerschaft von Patent- und Rechtsanwälten mbB
(86) Internationale Anmeldenummer: PCT/EP2009/059344
(87) Internationale Veröffentlichungsnummer: WO 2010/010082

(56) Entgegenhaltungen:
- EP-A2- 0 870 479
- WO-A1-02/09612
- WO-A1-2008/083358
- US-A- 4 970 032

## Beschreibung

Die vorliegende Erfindung betrifft einen Formkörper aus formstabilisiertem Material sowie ein Verfahren zu seiner Herstellung. Der erfindungsgemäße Formkörper ist insbesondere zum Einsatz in der Dentaltechnik bestimmt.

Die Herstellung von Zahnersatz erfolgt zunehmend maschinell, wobei bspw. das CAD/CAM-Verfahren oder das Kopierfräsverfahren zur Anwendung kommen. Dabei werden zum Beispiel aus einem porösen oder dichten keramischen Formkörper oder aus einem Kunststoffformkörper die Zahnrestauration, bspw. eine Zahnkrone, ein Inlay, ein Onlay, ein Veneer oder ein entsprechendes Gerüst, herausgearbeitet.

Um ästhetische Ansprüche zu befriedigen und Zahnrestaurationen mit einer Anmutung zu erzielen, die der natürlicher Zähne möglichst nahe kommt, werden mehrfarbige Formkörper hergestellt.

Die EP-A-455 854 offenbart zum Beispiel einen keramischen Formkörper aus üblichem Keramik- oder Porzellanmaterial mit mehreren farblich unterschiedlichen Schichten. Die Schichten können von glasartig durchscheinend im okklusalen Bereich bis gelblich opak im zervikalen Bereich ausgebildet sein.

Der Nachteil des in der EP-A-455 854 beschriebenen schichtweisen/planaren Aufbaus des Keramikkörpers ist, dass mit diesem zwar in vertikaler Richtung, von okklusal nach zervikal, ein dem natürlichen Zahn ähnlicher Farbverlauf generiert werden kann, nicht jedoch in horizontaler Richtung, was besonders im Frontzahnbereich von entscheidender Bedeutung für die Ästhetik ist. Zudem ist die Möglichkeit des Farbverlaufs der Restauration durch die bereits bei der Produktion eingestellte Dicke/Dimension der Schichten beschränkt, so dass der Anwender in der Verwendung eines solchen Blockes eingeschränkt ist oder eine Vielzahl von Blöcken mit unterschiedlichen Schichtdimensionen hergestellt werden müssen, um dem natürlichen Farbverlauf verschiedener Zähne gerecht zu werden.

Die EP-A-870 479 offenbart, dass der Farbverlauf einer Zahnrestauration verbessert und entsprechend den hohen Anforderungen an die Ästhetik des Zahnfarbbildes unabhängig vom Ausgangsmaterial eingestellt werden kann, wenn verschiedene Ausgangsmaterialien miteinander verpresst werden. Die Kontaktflächen werden mit Druck beaufschlagt und die unterschiedlich gefärbten Ausgangsmaterialien werden im Grenzbereich in innigen Kontakt gebracht, so dass dort eine - wenn auch geringe - Vermischung stattfindet. Infolge dieser Vermischung, welche durch die Form und Größe der Teilchen der Ausgangsmaterialien und/oder durch den Pressdruck beeinflusst werden kann, ist ein fließender Farbverlauf erzielbar und der tatsächliche Grenzverlauf zwischen den Ausgangsmaterialien ist nicht ersichtlich. Andererseits können im Wege des Pressverfahrens optional auch deutlich gegeneinander abgegrenzte Farbbereiche erzeugt werden. Durch die Anwendung des Pressverfahrens können bei niedrigen Kosten infolge geringeren Vorbereitungsaufwandes sowohl Kunststoff als auch Keramik als Ausgangsmaterialien eingesetzt werden.

In der EP-A-870 479 wird der Herstellungsprozess für planar geschichtete Blöcke beschrieben, wobei die Schichtung horizontal und/oder vertikal verlaufen kann. Generell gilt es zu beachten, dass der Übergang der Farbschichten auf der Restauration sehr hart und deutlich ist, wenn die Restauration aus geschichteten Blöcken geschliffen wurde und die Grenzflächen der Farbschichten an der Restaurationsoberfläche austreten. Trotz der beschriebenen Vermischung ist der Grenzflächenverlauf zwischen den Schichten erkennbar. Dies gilt auch für den Gegenstnd der zuvor beschriebenen Patentanmeldungen.

Ein weiterer Nachteil des in der EP-A-870 479 beschriebenen schichtweisen/planaren Aufbaus des Keramikkörpers ist, dass mit diesem nur in eine Richtung ein Farbverlauf generiert werden kann. Bei horizontaler Schichtung im Block liegt ein Farbverlauf in vertikaler Richtung in der Restauration vor, d.h. von okklusal nach zervikal, wodurch in dieser Richtung ein dem natürlichen Zahn ähnlicher Farbverlauf generiert werden kann, nicht jedoch senkrecht hierzu, d.h. in horizontaler Richtung, was besonders im Frontzahnbereich von erheblicher Bedeutung für eine ästhetische Anmutung ist.

Bei vertikaler Schichtung kann bei entsprechender Positionierung der zu schleifenden Restauration im Block ein einem Frontzahn ähnlicher Farbverlauf bzgl. des transluzenten Schmelzes in Form eines Bogens generiert werden, allerdings nur in eingeschränktem Maße, da der Verlauf von den Schichtdimensionen und Positionierung abhängig ist und somit für verschiedene Fälle eine Vielzahl an Blöcken mit unterschiedlich dicken Schichten bereitgestellt werden müssen. Darüber hinaus ist ein dem natürlichen Zahn äquivalenter Farbverlauf von okklusal nach zervikal nur eingeschränkt darstellbar.

Die WO-A-02/09612 offenbart eine Dentalprothese, umfassend eine Plattform, die daran angepasst ist an eine Fräsmaschine angeschlossen zu sein und ein Werkstück, das auf die Plattform montiert werden kann. Das Werkstück weist eine Vielzahl von Farbabstufungen auf, die unterschiedlichen Farbdichten natürlicher Zähne oder Teilen davon entsprechen, wobei das Werkstück nach Bearbeitung als Zahnersatz dient. Die Farbabstufungen sollen dabei allmählich verlaufen, wie es dem Farbverlauf natürlicher Zähne entspricht, von einer helleren Farbe zu einer dunkleren Farbe hin, wobei die helle Farbe dem Zahnschmelz und die dunklere Farbe dem Dentin entspricht. Interessant ist, dass diese Druckschrift bereits versucht den natürlichen Verlauf der Dentin/Schmelzgrenze nach zu ahmen. Der Fachmann ist mithin bekannt was unter dem natürlichen Verlauf der Dentin/Schmelzgrenze zu verstehen ist und daher keineswegs ein unklarer Begriff.

WO-A-2008/083358 offenbart einen Rohling für die Herstellung von Zahnersatz, der in einer äußeren Zone eine andere Farbe aufweist als in einer inneren Zone.

Der Nachteil des in der WO-A-2008/083358 beschriebenen Rohlings ist, dass die inneren Schichten geometrische Formen, Zylinder und eine rechteckige Form, aufweisen, die konzentrisch mit den äußeren Schichten angeordnet sind. Hierdurch wird die Anzahl an Möglichkeiten zur Nachbildung des natürlichen Farb- und Transluzenzverlaufs von Zähnen eingeschränkt bzw. die mögliche Ästhetik der Restauration ist zwar höher als bei Restaurationen aus planar geschichteten Blöcken, erreicht aber nicht das natürliche Vorbild. Es wird auch nur eine vage Beschreibung der Form der verschiedenen Zonen im Block gegeben.

Die offenbarte Schichtung mit mehr als 2 Schichten weist die gleichen Nachteile auf, wie die Blöcke, die in EP-A-455 854 und US-A-4,970,032 offenbart werden. Durch die produktionstechnisch bedingten Schichtdicken ist die Zahl möglicher Restaurationen begrenzt.

Die US-A-4 970 032 betrifft einen mehrfarbigen Kunststoffformkörper mit vorgegeben variierenden Farbschichten, die übereinander um einen Kern angeordnet sind. Zur Herstellung des Kerns und der Schichten wird ein Spritzformgebungsverfahren angewendet.

US-A-4,970,032 offenbart auch ein Verfahren zur Herstellung von künstlichen Zähnen entweder durch Ersatz des gesamten Zahnes oder Kronen, wobei der Zahn hergestellt werden kann durch maschinelle Bearbeitung eines geschichteten Blocks aus synthetischem Kunststoffmaterial, wobei der Block Schichten aufweist mit vorher festgelegter Farbe, Farbanmutung und Transluzenz um Pulpa, Dentin und Schmelz eines natürlichen Zahns nachzuahmen und dabei einen künstlichen Zahn zur Verfügung zu stellen, der die Farbverteilung und Farbtiefe eines natürlichen Zahns besitzt.

Der in der US-A-4,970,032 beschriebene Kunststoff-Block weist einen zylindrischen Innenkörper auf, der von mindestens zwei Schichten umgeben ist. Es liegen somit mindestens drei Schichten vor. Der Erfindung liegt der Gedanke zu Grunde, dass die bei natürlichen Zähnen auftretenden Farbverläufe durch unterschiedlich eingefärbte Schichten im Block nachgebildet werden sollen. Der Nachteil daran ist jedoch, dass die bei natürlichen Zähnen mannigfachen Größen/Dimensionen der Farbschichten nicht in einem Block nachgebildet werden können. Durch die bei der Produktion festgelegten Dimensionen der Schichten sowie ihren Verlauf um den zylindrischen Innenkörper ist die Anwendung des Blockes eingeschränkt, da die erforderlichen Farbverläufe der herzustellenden Restauration trotz unterschiedlicher Positionierung der Restauration im Block nicht zwingend abgebildet werden können. Hierdurch ist die Herstellung mehrerer Blöcke mit unterschiedlich dimensionierten Schichten sowie Schichtverläufen um den zylindrischen Kern notwendig. Besonders für Frontzahnrestaurationen ist dieses zu beachten.

Ein der Erfindung zugrunde liegendes technisches Problem kann darin gesehen werden, einen Formkörper zu schaffen, der eine verbesserte Anmutung einer fertigen Zahnrestauration ermöglicht und insbesondere eine Vielzahl von Anordnungs- und Gestaltungsmöglichkeiten des Dentin/Schmelzgrenzverlaufs ermöglicht. Des Weiteren soll die Erfindung ein Verfahren angeben, dass es ermöglicht den Formkörper herzustellen.

Erfindungsgemäß werden die technischen Probleme gelöst durch einen Formkörper aus formstabilisiertem Material mit den Merkmalen des Anspruchs 1.

'Der erfindungsgemäße Formkörper ist in einer Ausführungsform so ausgebildet, dass die Grenzfläche zumindest teilweise dem Verlauf der Dentin/Schmelzgrenze natürlicher oder künstlicher Zähne entspricht. Dabei werden die Färbungen des ersten Bestandteils und zweiten Bestandteils so gewählt, dass sie der Schmelz- bzw. Dentinfärbung eines natürlichen Zahns oder Kunstzahns möglichst nahe kommen. Dadurch wird es möglich, eine Vielzahl von Verläufen der Dentin/Schmelzgrenzen von Zähnen in dem Formkörper vorzusehen, so dass eine individuelle Anpassung der Grenzverläufe in einer anzufertigenden Zahnrestauration erfolgen kann.

Der erfindungsgemäße Formkörper kann durch verschiedene Maßnahmen in seiner Form stabilisiert werden. Insbesondere kann dies durch mechanische Einwirkung, insbesondere durch Druck, typischerweise im Bereich von 5-500 MPa, zum Beispiel Pressen, bewerkstelligt werden. Weitere Formgebungsverfahren sind dem Fachmann bekannt. Zudem können aufbauende Verfahren, z.B. Injection Moulding, zur Herstellung verwendet werden.

Die Färbung der Bestandteile des erfindungsgemäßen Formkörpers kann durch Pigmentierung, im einfachsten Fall mittels Farbpigmenten erfolgen, die auch gleichzeitig der Einstellung einer Transluzenz dienen kann. Die Transluzenz kann auch durch Trübungsmittel eingestellt werden.

Für den Einsatz des erfindungsgemäßen Formkörpers in der Dentaltechnik eignen sich insbesondere solche Formkörper, deren erster und zweiter Bestandteil eine Keramik, insbesondere eine Feldspat- oder Oxidkeramik ist. Es ist aber genau so möglich ein Kunststoffmaterial einzusetzen. Der Kunststoff kann ein thermoplastischer Kunststoff oder duroplastischer Kunststoff sein. Insbesondere geeignet ist ein Kunststoff auf Acrylatbasis. Geeignete Kunststoffe sind dem Fachmann bekannt und sind typischerweise solche, die bei der Herstellung von Kunstzähnen Verwendung finden.

Es kommen auch erste und zweite Bestandteile aus einer Feldspatkeramik in Betracht, die Metalloxide umfasst, die ausgewählt sind aus der Gruppe bestehend aus SiO₂, Al₂O₃, Na₂O, K₂O, gegebenenfalls ergänzt durch Zugabe von Pigmenten und anorganischen Füllstoffen.

In einer weiteren Ausführungsform können die Bestandteile aus einer Oxidkeramik aufgebaut sein, die Metalloxide umfasst, die ausgewählt sind aus der Gruppe bestehend aus SiO₂, Al₂O₃, ZrO₂, stabilisiert durch verschiedene Verbindungen (Y₂O₃, CeO₂, etc.), gegebenenfalls ergänzt durch Zugabe von Pigmenten bzw. Verbindungen färbender Ionen.

Bei den beiden beschriebenen Ausführungsformen aus Feldspatkeramik und Oxidkeramiken kann der Formkörper sowohl porös gesintert werden, ehe er weiterverarbeitet wird, oder aber dicht gesintert werden. Hierbei gilt es, die verschiedenen Anwendungsmöglichkeiten zu unterscheiden, wovon einige exemplarisch genannt werden:
Aus einem dicht gesinterten Feldspatblock kann z. B. eine Vollkrone geschliffen oder gefräst werden, aber auch Inlays, Onlays oder Veneers. Zudem sind bereits Techniken bekannt, bei denen aus dichten Feldspat- oder Glaskeramikblöcken Verblendschalen herausgeschliffen werden, die mittels Glaslot auf ein Gerüst aufgesintert werden oder mit Hilfe eines organischen Klebers aufgeklebt werden. Zudem können diese dichten Verblendschalen aufgesintert werden.

Aus einem porösen Feldspatmaterial lässt sich z.B. sowohl eine Krone herausschleifen, wobei der Schrumpf des porösen Materials, der beim dicht-Sintern auftritt und richtungsabhängig sein kann, bei der Generierung der Schleifdaten berücksichtigt werden muss, als auch eine sogenannte Verblendschale, die im porösen Zustand auf ein Gerüst aufgesetzt wird und anschließend z.B. aufgesintert wird. Die Restauration bzw. Zwischenprodukte der endgültigen Restauration werden beim Schleifen aus einem porösen Block jeweils vergrößert herausgeschliffen. Beim Herausschleifen der porösen Verblendschale muss allerdings nicht, wie bei anderen Verfahren, z.B. der Herstellung von Gerüsten, bereits üblich, der Vergrößerungsfaktor auf die gesamte Verblendschale angewendet werden, sondern es ist zur Vermeidung von Hohlräumen vorteilhaft, wenn die die Innenkontur der Verblendschale und die Oberfläche des Gerüstes derartig gestaltet werden, dass sie möglichst ein Form und eine Gegenform bilden und der Vergrößerungsfaktor nicht linear auf die Verblendschale angewendet wird.

Aus einem porösen Oxidkeramikblock kann z.B. ein Gerüst herausgeschliffen werden, wobei durch die entsprechende Positionierung des Gerüstes im mehrfarbigen Block ein Farbverlauf im Gerüst geschaffen wird. Nach dem Schleifen wird das Gerüst, wenn es zuvor vergrößert geschliffen wurde, dicht gesintert. Im Falle einer Infiltrationskeramik kann das z.B. Gerüst im Maßstab 1:1 geschliffen und anschließend mit Glas infiltriert werden.

In einer anderen Ausführungsform der Erfindung enthält der erfindungsgemäße Formkörper einen Binder zur Verbesserung der Formstabilität insbesondere des sinterfähigen Materials. Ein einsetzbarer Binder ist zum Beispiel ausgewählt aus der Gruppe bestehend aus Acrylat (-en), Polyvinylalkohol (PVA), Polyvinylacetat (PVAC), Polysaccharid/Acrylsäure (PS/AC), Cellulosederivate oder Mischungen davon. Zusätzliche Hilfstoffe wie Wasser, Schmiermittel zur Erniedrigung der Reibung, Sinterhilfsmittel zur Beschleunigung der Verdichtung oder Dispergiermittel, Plastifizierer, Benetzungsmittel und Thermoplaste zur Beeinflussung der rheologischen Eigenschaften können zugegeben werden.

Erfindungsgemäß ist insbesondere vorteilhaft, dass der zweite Bestandteil des Formkörpers in dem ersten Bestandteil angeordnet ist. Der erste Bestandteil umgibt den zweiten Bestandteil somit zumindest in Bereichen, die nach dem Einsetzen der Restauration in den Mund des Patienten sichtbar sind. Der zweite bzw. innere Bestandteil des Formkörpers ist somit allenfalls vor dem Einsetzen in den Mund des Patienten in Bereichen sichtbar, die nach dem Einsetzen in Richtung des Kiefers weisen. Der zweite Bestandteil ist somit von dem ersten Bestandteil zumindest in dem später sichtbaren Bereich umhüllt. Die Grenzfläche zwischen den beiden Bestandteilen ist somit bei eingesetztem Formkörper nicht sichtbar. Eine Schichtung kann beim eingesetzten Formkörper nicht festgestellt werden. Insbesondere sind keine Schichtgrenzen erkennbar.

Die erfindungsgemäße Ausgestaltung des Formkörpers hat ferner den Vorteil, dass unterschiedliche Zähne bzw. Zahnformen im Raum so in den Formkörper hineingelegt werden können, dass stets ein Formkörper entsteht, bei dem die Grenzfläche zwischen den beiden Bestandteilen innerhalb des Formkörpers wie vorstehend angeordnet ist. Mit Hilfe eines einzigen erfindungsgemäßen Formkörpers ist es somit möglich, eine Vielzahl von unterschiedlichen Zähnen bzw. Zahnformen nachzubilden.

Die Grenzfläche zwischen dem ersten und zweiten Bestandteil des Formkörpers, d.h. insbesondere die Grenzfläche zwischen dem das Dentin bzw. den Schmelz eines Zahnes nachbildenden Materials ist im Wesentlichen durch eine Parabelschar beschrieben. Zueinander parallele Querschnittsebenen durch den Formkörper können derart in den Formkörper gelegt werden, dass die Grenze zwischen den beiden Bestandteilen als parabelförmige Grenzlinie erscheint. Hierbei ist die Grenzlinie zumindest über 2/3, insbesondere 3/4, ihrer Länge parabelförmig. Insbesondere die

Ränder bzw. Enden der Grenzlinie können eine von einer Parabel abweichende Form aufweisen, wobei auch dieser Bereich der Grenzlinie vorzugsweise keinen Sprung oder keine Stufe aufweist. Insbesondere wird die parabelförmige Grenzlinie in den Randbereichen flacher und ist vorzugsweise nach außen gerichtet.

Über eine Breite des Formkörpers, die sich senkrecht zu den parallelen Querschnittsebenen erstreckt, weisen mindestens 70% der Querschnittsebenen, insbesondere mindestens 80% der Querschnittsebenen eine parabelförmige Grenzlinie, wie vorstehend definiert auf.

Bei einer Ausrichtung der Formkörper, bei der der zweite Bestandteil, d.h. insbesondere, das das Dentin nachbildendende Material im unteren Bereich des Formkörpers angeordnet ist, sind die die Grenzlinien ausbildenden Parabeln nach unten offen. Hierdurch werden Maxima ausgebildet, wobei in bevorzugter Ausführungsform die Parabeln bezüglich einer durch die Maxima verlaufenden Ebene spiegelsymmetrisch sind. Bei dieser Anordnung des Formkörpers mit nach unten weisendem Dentin, kann somit eine Symmetrieebene durch sämtliche Maxima der Parabeln gelegt werden.

In bevorzugter Ausführungsform kann eine Haupt-Querschnittsebene definiert werden, bei der es sich in der Lage des Formkörpers mit nach unten weisenden Dentin um diejenige Querschnittsebene handelt, in der die parabelförmige Grenzlinie das größte bzw. höchste Maximum aufweist. Ausgehend von der Haupt-Querschnittsebene in eine Verjüngungsrichtung nimmt die Höhe der Grenzlinie vorzugsweise stetig ab. Insbesondere eine durch die Maxima definierte Kurve nimmt zumindest über einen Großteil ihrer Länge von insbesondere mehr als die Hälfte und vorzugsweise mehr als 3/4 ihrer Länge stetig ab. Die Verbindungskurve der Maxima liegt vorzugsweise in der Symmetrieebene und/oder ist senkrecht zur Haupt-Querschnittsebene ausgerichtet. Bezogen auf die Breite des zweiten Bestandteils in Verjüngungsrichtung erfolgt die Abnahme der Parabelmaxima erfindungsgemäß über mindestens 50%, besonders bevorzugt über mindestens 75% der Gesamtlänge bzw. Gesamtbreite des zweiten Bestandteils.

Vorzugsweise gehen beide Enden der Grenzlinie bzw. beide Enden der ParabelÄste in eine Kurve entgegengesetzter Krümmung über. An den parabelförmigen Teil der Grenzlinie schließt sich somit in bevorzugter Ausführungsform eine nach außen gekrümmte Kurve an, so dass ein Wendepunkt ausgebildet ist.

Die vorstehend beschriebene Ausführungsform der Grenzfläche zwischen dem ersten Bestandteil, der insbesondere den Schmelz nachbildet und dem zweiten Bestandteil, der insbesondere das Dentin nachbildet, kann geringe Abweichungen aufweisen. Es handelt sich hierbei um eine geometrische Annäherung, so dass Abweichungen von bis zu 10% möglich sind. Insbesondere erfolgt die Ausgestaltung der Grenzlinie anhand von Erfahrungswerten sowie anhand von Untersuchungen der Schmelz-Dentin-Grenze bei natürlichen und/ oder künstlichen Zähnen.

Zur Herstellung eines künstlichen Zahnes ist der Formkörper vorzugsweise über eine geeignete Computersoftware darstellbar und insbesondere im Raum frei drehbar. Ein Zahnarzt oder Zahntechniker kann somit die Grenzfläche aus unterschiedlichen Blickwinkeln betrachten. Beispielsweise über Bildverarbeitungsprogramme werden dem Rechner die geometrischen Abmessungen des nachzubildenden Zahnes übermittelt. Der Zahnarzt oder Zahntechniker kann den hierdurch erzeugten virtuellen Zahn beliebig in dem Formkörper anordnen und hierdurch die Grenzfläche zwischen Dentin und Zahnschmelz derart anordnen, dass der später aus dem Formkörper hergestellte Zahn im Erscheinungsbild bzw. der Anmutung den natürlichen Zähnen des Patienten, insbesondere den benachbarten Zähnen des Patienten entspricht oder zumindest sehr ähnlich ist. Durch den Bogenverlauf in Kombination mit der freien Positionierung der Restauration im Block ist nicht nur eine (spiegel-) symmetrische Gestaltung der Schmelz-Dentin-Grenze möglich, sondern auch eine horizontal asymmetrische Form, wie es bei natürlichen Zähnen üblich ist. Zudem kann durch die Positionierung des Restauration im Block eine sich von okklusal nach zervikal verjüngende Schicht Schmelzmasse über die Dentinmasse gelegt werden, wodurch ein Farbverlauf von Hell zu dunkel generiert wird, der keine horizontal verlaufende Schichtgrenzen aufweist und dem natürlichen, nicht abgestuften Farbverlauf entspricht.

Zur Herstellung des erfindungsgemäßen Formkörpers aus insbesondere sinterfähigem Material oder Kunststoffmaterial der mindestens einen ersten und mindestens einen zweiten Bestandteil aufweist, kann das erfindungsgemäße Verfahren dienen, bei dem
a) der mindestens erste Bestandteil in eine Form eingefüllt wird,
b) in den eingefüllten mindestens ersten Bestandteil des insbesondere sinterfähigen Materials oder Kunststoffmaterials eine Mulde mit einer Oberfläche eingepresst und
c) die Oberfläche eine räumlich gekrümmte Grenzfläche mit dem
d) in die Mulde eingefüllten mindestens zweiten Bestandteils bildet.

Im Falle der Verwendung eines Kunststoffmaterials des ersten und zweiten Bestandteils wird dieses insbesondere unter Temperatur und gegebenenfalls Druck ausgehärtet.

Die Oberfläche der Grenzfläche ist so gestaltet, dass sie zumindest teilweise dem Verlauf der Dentin/Schmelzgrenze natürlicher oder künstlicher Zähne entspricht.

Die Herstellung des Formkörpers kann auch durch keramisches Spritzgießen o.ä. Herstellungsverfahren erfolgen.

Die Konstruktion der Grenzfläche erfolgt gemäß einer Ausführungsform der Erfindung durch Positionierung der Schmelz-Dentin-Grenzflächen unterschiedlicher natürlicher und/oder künstlicher Zähne. Zur Charakterisierung der Lage der Grenzfläche von Schmelz und Dentin kann bei natürlichen Zähnen der Schmelz präparativ vorsichtig abgetragen werden. Dabei ist darauf zu achten, dass die Dentinschicht nicht abgetragen wird. Einfacher ist die Herstellung von künstlichen Zähnen und deren Grenzschichtverlauf. Der Grenzschichtverlauf bei künstlichen Zähnen ist dem Verlauf der natürlichen Zähne nachempfunden. Um Körper, die den Grenzschichtverlauf aufweisen, herzustellen, werden die Zähne nicht mit allen Schichten hergestellt, sondern die transluzentere Schmelzschicht wird nicht aufgetragen. Nach dem Sinterprozess erhält man so eine Oberfläche des künstlichen Zahns, die dem Grenzverlauf Schmelz zu Dentin entspricht.

Durch Präparation verschiedener Zähne kann man durch die Anordnung der verschiedenen Dentin-Schmelz-Grenzflächen im Raum, vorzugweise durch größenabhängige Anordnung, eine gekrümmte Fläche modellieren, die dem Grenzflächenverlauf der verschiedenen Zähne entspricht. Aus diesem Modell kann ein Werkzeug hergestellt werden.

Eine alternative Möglichkeit der Grenzflächenmodellierung ist, Schmelz-Dentin-Verläufe zu digitalisieren und die Modellierung über eine Software zu generieren. Dies bietet die Möglichkeit, eine wesentlich größere Zahl an Grenzverläufen in die Generierung der Grenzfläche einfließen zu lassen. Zudem kann ein Werkzeug mittels CAD-CAM-Verfahren dann einfacher hergestellt werden.

Mit Hilfe des Werkzeuges lässt sich ein gewünschter Formkörper herstellen. Um eine dreidimensionale Vermessung des Formkörpers bzw. des Grenzflächenverlaufes vorzunehmen, wird ein Formkörper in eine möglichst große Anzahl an planparallelen Scheiben gesägt und die Scheiben werden beidseitig glatt geschliffen. Dabei wird vor dem Zersägen senkrecht zur Sägerichtung eine durchgehende Markierung an dem Formkörper angebracht, vorzugsweise eine Nut, die diagonale Ecken des Formkörpers verbindet, die eine eindeutige Lagebestimmung einer Scheibe durch Vermessen der Position Nut an der Seite der Scheibe ermöglicht.

Durch das Vermessen der Lage der Grenzfläche auf den Scheiben lässt sich in einem Koordinatensystem eine Punktwolke erstellen, die die Grenzfläche Schmelz-Dentin darstellt.

Im erfindungsgemäßen Verfahren kann als Zwischenschritt einen Sinterschritt anschließen. Dies hat den Vorteil, dass der Formkörper stabiler ist.

Der erfindungsgemäße Formkörper kann auch durch entsprechend ausgeführte Biskuitierung- oder Schlickerverfahren wie beispielsweise Schlickerdruckguss hergestellt werden.

Der Herstellprozess mit Hilfe von Schlickerdruckguss kann beispielsweise folgendermaßen durchgeführt werden:
a.) Herstellung eines Formteils A mit der Geometrie der Oberfläche aus dem o.g. Herstellprozess durch Eingießen eines Schlickers in bspw. eine poröse Gipsform, deren Oberfläche der beschriebenen Grenze entspricht unter Anwendung von Druck oder ohne Druck
b.) Herstellung eines zweiten Formteils B mit Negativkontur gegenüber Formteil A gemäß a.)
c.) Fügen der beiden Formteile A und B zu einer Gesamteinheit C mit möglicher thermischer Nachbehandlung mit oder ohne Druck innerhalb oder außerhalb einer Form (nach Entformung), welche die Gesamtaußenform der Gesamteinheit C bestimmt (Oberfläche des gefügten Bauteils/Gesamteinheit)
Oder:
a.) Herstellung eines Formteils A mit der Geometrie der Oberfläche aus dem o.g. Herstellprozess durch Eingießen eines Schlickers in bspw. eine poröse Gipsform, deren Oberfläche der beschriebenen Grenze entspricht unter Anwendung von Druck oder ohne Druck
b.) Entformung des Formteils A und Positionierung des Formteils A in einer weiteren Gipsform, die der Endkontur der Gesamteinheit entsprechen soll.
c.) Auffüllen der Form mit einem weiteren Schlicker unter Anwendung von Druck oder ohne Druck und möglicher thermischer Nachbehandlung innerhalb der Form oder nach Entformung.

Der Herstellprozess mit Hilfe von Bisquitierung kann folgendermaßen durchgeführt werden:
a.) Herstellung eines Formteils A mit der Geometrie der Oberfläche aus dem o.g. Herstellprozesses durch Einlegen einer plastifizierten Masse
   mit oder ohne Binderzusätzen in eine entsprechende Form und möglicher thermischer Stabilisierung mit oder ohne Druck
b.) Herstellung eines zweiten Formteils B mit Negativkontur gegenüber Formteil A gemäß a.)
c.) Fügen der beiden Formteile A und B zu einer Gesamteinheit C mit möglicher thermischer Nachbehandlung mit oder ohne Druck innerhalb oder außerhalb einer Form, welche die Gesamtaußenform der Gesamteinheit C bestimmt (Oberfläche des gefügten Bauteils/Gesamteinheit).
Die beschriebenen Herstellverfahren sind in der Keramikformgebung prinzipiell schon lange bekannt (Hülsenberg, Keramikformgebung, ISBN 3--342-00098-8)

Der erfindungsgemäße Formkörper kann zur Herstellung einer Dentalrestauration eingesetzt werden, die insbesondere durch CAD/CAM-Methoden konstruiert und hergestellt wird.

Offenbart ist auch ein Verfahren zur Herstellung eines Formkörpers aus formstabilisiertem Material mit einer im Formkörper verlaufenden Grenzfläche, wobei das Material mindestens einen ersten Bestandteil und einen zweiten Bestandteil aufweist,
der zweite Bestandteil eine andere Pigmentierung als der erste Bestandteil aufweist und der zweite Bestandteil in dem ersten Bestandteil unter Ausbildung einer Grenzfläche so angeordnet ist, dass die Grenzfläche eine räumlich gekrümmte Fläche darstellt,
die Grenzfläche erhältlich ist, indem Flächen, die Krümmungsradien mit unterschiedlichen Krümmungsgraden besitzen, aus Schnitten, die durch ein Ensemble natürlicher oder künstlicher Zähne geführt sind, geschaffen werden und/ oder
die Grenzfläche erhältlich ist, indem Flächen, die Krümmungsradien mit unterschiedlichen Krümmungsgraden besitzen, aus Verläufen der Dentin-Schmelzgrenze natürlicher oder künstlicher Zähne des Ensembles geschaffen werden,
die geschaffenen Flächen mit Krümmungsradien mit unterschiedlichen Krümmungsgraden, in Abhängigkeit des Krümmungsgrades der Krümmungsradien räumlich angeordnet werden, und
bei welcher sich durch eine sich dadurch ergebende räumliche Anordnung der geschaffenen Flächen die Gesamtheit der Grenzfläche ergibt.

In einer Ausführungsform des offenbarten Verfahrens zur Herstellung des erfindungsgemäßen Formkörpers mit Grenzfläche kann beim Anordnen der geschaffenen Flächen der Randbereich der Verläufe der Dentin-Schmelzgrenze unberücksichtigt bleiben.

In einer anderen offenbarten Ausführungsform kann das Ausbilden der Gesamtheit der Grenzfläche nur Zähne berücksichtigen, deren Dentin-Schmelzgrenze mit einer vorgegebenen Näherungsfläche zumindest 80%, insbesondere zumindest 90% übereinstimmt. Dabei wird insbesondere die Näherungsfläche durch signifikante Dentin-Schmelzgrenzen natürlicher oder künstlicher Zähne angenähert.

In noch einer Ausführungsform des offenbarten Verfahrens werden die Zähne mit starker Krümmung der Dentin-Schmelzgrenze zur Ausbildung eines Scheitelbereichs der Grenzfläche zusammengefasst, bzw. im Scheitelbereich einer Näherungsfläche angeordnet. Die zur Anordnung im Scheitelbereich ausgewählten Zähne können zum Beispiel im Wesentlichen der Größe nach sortiert werden.

In einer weiteren Ausführungsform des offenbarten Verfahrens werden die Zähne mit schwacher Krümmung der Dentin-Schmelzgrenze im Randbereich der Grenzfläche zusammengefasst, bzw. im Randbereich einer Näherungsfläche angeordnet. Auch hier können die zur Anordnung im Randbereich ausgewählten Zähne im Wesentlichen der Größe nach sortiert werden.

In einer anderen Ausführungsform des offenbarten Verfahrens können die Zähne mit mittlerer Krümmung der Dentin-Schmelzgrenze zur Ausbildung eines zwischen dem Randbereich und dem Scheitelbereich angeordnetem Zwischenbereichs der Grenzfläche zusammengefasst, bzw. im Zwischenbereich einer Näherungsfläche angeordnet werden. Die zur Anordnung im Zwischenbereich ausgewählten Zähne können auch hier im Wesentlichen der Größe nach sortiert werden.

Erfindungsgemäß ist es ebenfalls möglich, dass die Größensortierung unabhängig von der Stärke der Krümmung in dieselbe Raumrichtung erfolgt.

Nachfolgend ist ein erfindungsgemäßer Formkörper in bevorzugten Ausführungsformen dargestellt.

Es zeigen:
Figur 1 eine schematische Seitenansicht eines Formkörpers,
Figur 2 eine schematische Rückansicht des in Figur 1 dargestellten Formkörpers in Richtung des Pfeils II,
Figur 3 eine schematische Schnittansicht entlang der Linie III-III in Figur 2,
Figur 4 Darstellungen der Grenzlinie in unterschiedlichen in Figur 3 definierten Querschnittsebenen,
Figur 5 eine bevorzugte Ausführungsform eines erfindungsgemäßen Formkörpers mit einer schematischen Darstellung des herzustellenden künstlichen Zahnes in Vorderansicht,
Figur 6 der in Figur 5 dargestellte Formkörper in Seitenansicht, wobei der Zahn schematisch und durchsichtig dargestellt ist,
Figur 7 eine der Figur 6 entsprechende Seitenansicht, wobei der Dentinbestandteil des Formkörpers schematisch und durchsichtig dargestellt ist,
Figur 8 eine Rückansicht des in Figur 5 dargestellten Formkörpers,
Figur 9 eine dreidimensionale Darstellung eines Koordinatensystems, in dem Untersuchungsergebnisse der Dentin-Schmelzgrenze natürlicher und künstlicher Zähne angegeben ist,
Figur 10 eine Projektion der in Figur 9 erfolgten Darstellung in die X-Y-Ebene,
Figur 11 eine Projektion der in Figur 9 erfolgten Darstellung in die Y-Z-Ebene und
Figur 12 eine Darstellung der Untersuchungsergebnisse entsprechend Figur 9 mit veränderter Ausrichtung des Koordinatensystems.

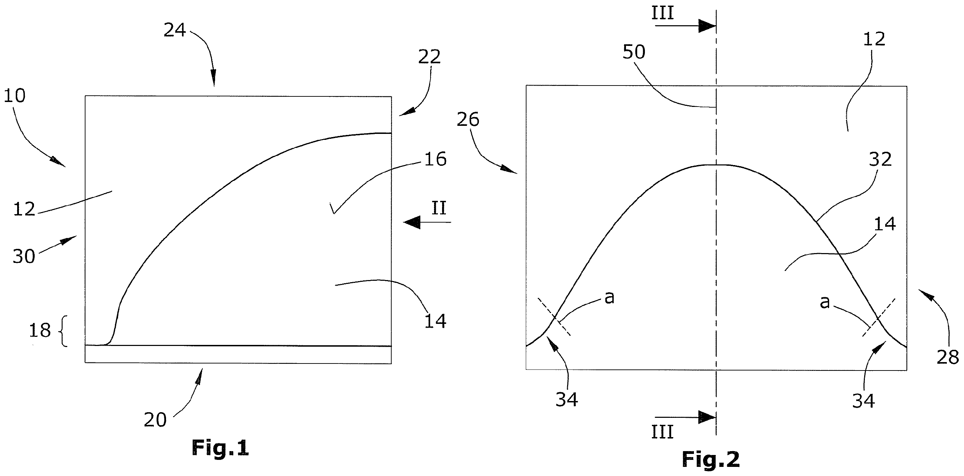

Im Folgenden wird anhand der Figuren 1 - 4 die der erfindungsgemäßen Ausgestaltung des Formkörpers zugrundeliegende geometrische Ausgestaltung der Grenzfläche beschrieben.

Ein Formkörper 10 weist einen ersten Bestandteil 12 und einen zweiten Bestandteil 14 auf. Bei dem ersten Bestandteil 12 handelt es sich um das den Schmelz eines Zahnes nachbildende Material. Bei dem zweiten Bestandteil 14 handelt es sich um das das Dentin eines Zahnes nachbildende Material. Erfindungsgemäß ist eine Grenzfläche 16 zwischen den beiden Bestandteilen 12, 14 als im Raum gekrümmte Grenzfläche ausgebildet. Die Grenzfläche 16 kann mathematisch zumindest näherungsweise durch eine Parabelschar beschrieben werden. Dies gilt zumindest für einen Großteil der Querschnittsfläche, wobei insbesondere in einem unteren Bereich 18 gegebenenfalls Abweichungen von der Parabelform gegeben sind.

Die Grenzfläche 16 ist derart in dem Formkörper 10 angeordnet, dass der zweite Bestandteil 14 im dargestellten Ausführungsbeispiel die gesamte Grenzfläche 16 des quaderförmigen Formkörpers ausbildet. In Rückansicht (Figur 2), in der die seitliche Außenfläche 22 des Formkörpers 10 sichtbar ist, ist ein Teil der Außenfläche durch den zweiten Bestandteil 14 und der übrige Teil durch den ersten Bestandteil 12 ausgebildet. Eine obere Außenfläche 24 des Formkörpers 10 ist ausschließlich durch den ersten Bestandteil 12 ausgebildet. Die beiden einander gegenüberliegenden Außenflächen 26, 28 (Figur 2) des Formkörpers sind zu einem sehr großen Bestandteil durch den ersten Bestandteil 12 ausgebildet. Nur im unteren Bereich sind die Außenflächen 26, 28 durch den zweiten Bestandteil 14 ausgebildet. Entsprechend ist auch die Außenfläche 30 (Figur 1) ausgebildet.

Aus der Rückansicht (Figur 2) ist eine Grenzlinie 32 der Grenzfläche 16 sichtbar. Die Grenzlinie 32, d.h. die Grenze zwischen den beiden Bestandteilen 12, 14 ist zumindest zwischen den beiden gestrichelt dargestellten Hilfslinien a parabelförmig ausgebildet. Im Anschluss an die parabelförmige Ausgestaltung schließen sich Enden 34 der Grenzlinie 32 an. Diese weisen einen nach außen gerichteten Verlauf und im dargestellten Ausführungsbeispiel eine Krümmungsänderung auf. In dem Endbereich 34 der Grenzlinie 32 ist somit ein Wendepunkt vorgesehen.

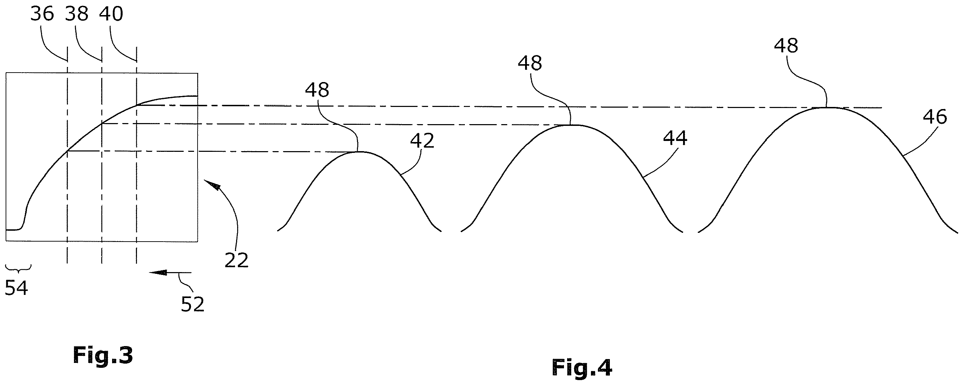

In unterschiedlichen Querschnittsebenen 36, 38, 40 (Figur 3) weist die Grenzfläche 16, Grenzlinien 42, 44, 46 (Figur 4) auf, die in ihrer mathematischen Grundstruktur der Kurve 32 (Figur 2) entsprechen. Die Grenzlinien 42, 44, 46 sind somit ebenfalls mit Ausnahme ihrer jeweiligen Enden parabelförmig ausgebildet. Die Enden weisen wiederum nach außen und bilden einen Wendepunkt.

Jede Parabel der einzelnen Querschnittsebenen 36, 38, 40 weist ein Maximum 48 auf. In dem in Figur 3 dargestellten Längsschnitt entlang einer Symmetrieebene 50 (Figur 2) ist die Kurve der Maxima sichtbar. Die Kurve der Maxima nimmt über eine Breite des Formkörpers 10 bzw. in Verjüngungsrichtung 52 stetig ab. Dies ist zumindest über einen Großteil der Gesamtlänge der Kurve der Maxima zutreffend. Gegebenenfalls ergeben sich Abweichungen in einem Endbereich 54. Im dargestellten Ausführungsbeispiel nehmen die Maxima der Parabeln, ausgehend von einer Haupt-Querschnittsebene ab, wobei im dargestellten Ausführungsbeispiel die Haupt-Querschnittsebene der Außenfläche 22 entspricht.

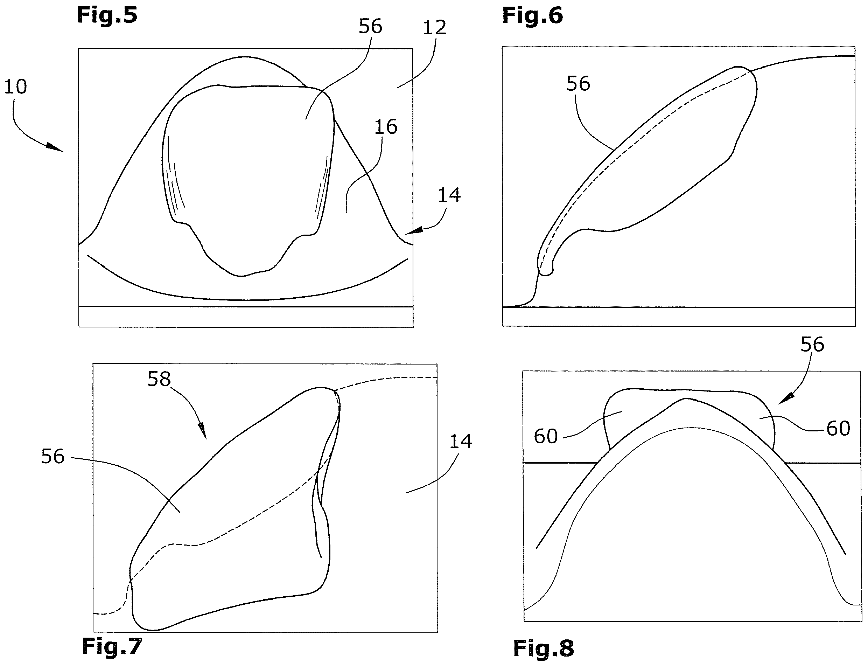

In den Figuren 5 - 8 ist eine bevorzugte Ausführungsform eines Formkörpers dargestellt, wobei es sich insbesondere um einen virtuellen, mit Hilfe einer Computersoftware dargestellten Formkörper handelt. Der Formkörper ist grundsätzlich, wie vorstehend anhand der Figuren 1-4 erläutert, aufgebaut und weist einen ersten Bestandteil 12 und einen zweiten Bestandteil 14 auf. Ein herzustellender künstlicher Zahn 56 kann virtuell in den Formkörper 10 hineingelegt werden. Es ist hierbei möglich, den Zahn beliebig in dem Formkörper 10 anzuordnen, so dass die Grenzfläche zwischen den beiden Bestandteilen 12, 14, d. h. die Dentin-Schmelz-Grenzfläche 16 in den Zahn 56 hineingelegt werden kann. Hierzu sind die Außenabmessungen des herzustellenden künstlichen Zahns 56 bekannt. Diese können beispielsweise aus vorgegebenen Mustern ausgewählt werden. Auch kann eine dreidimensionale Darstellung durch Aufnahme mehrerer Bilder des zu ersetzenden oder eines benachbarten Zahns erfolgen.

Gegebenenfalls können Kombinationen von virtuellen Zahnmustern mit über Bildverarbeitung erfassten geometrischen Daten erfolgen. Gegebenenfalls kann auch eine Bearbeitung der Form durch den Benutzer wie den Zahnarzt oder den Zahntechniker vorgenommen werden. Der herzustellende künstliche Zahn 56 kann, um eine möglichst naturgetreue Anmutung zu erlangen von dem Zahnarzt bzw. Zahntechniker in unterschiedlichen Lagen hinsichtlich der Grenzfläche angeordnet werden. Der erste Bestandteil 12 des Formkörpers, durch den der Schmelz nachgebildet wird, kann somit in unterschiedlichen Bereichen eine unterschiedliche Dicke aufweisen, so dass die Grenzfläche zwischen den beiden Bestandteilen innerhalb des herzustellenden Zahns 56 der Dentin-Schmelz-Grenze eines natürlichen Zahns des Patienten angenähert ist.

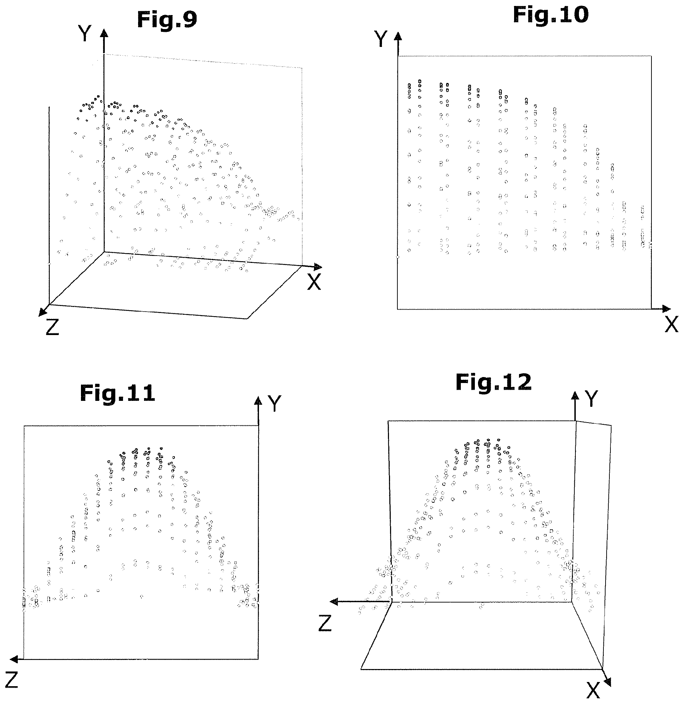

Zur Verdeutlichung der Lage des herzustellenden Zahns 56 ist dieser in Figur 6 durchsichtig dargestellt. Die Grenzlinie ist gepunktet dargestellt. Ferner ist in Figur 7 gegenüber dem das Dentin ausbildenden zweiten Bestandteil 14 gepunktet dargestellt. Hieraus wird ersichtlich, dass die gesamte spätere Vorderseite 58 des herzustellenden Zahns 56 durch den ersten Bestandteil 12, der den Schmelz nachbildet, ausgebildet sein wird. Ferner können, wie insbesondere aus Figur 8 ersichtlich ist, Bereiche des Zahns 56 ausschließlich aus dem ersten Bestandteil 12 bestehen. Es handelt sich hierbei um die Bereiche 60. Die insbesondere anhand der Figuren 1-4 erläuterte mathematische Annäherung basiert in bevorzugter Ausführungsform auf Untersuchungen, die im Folgenden anhand der Figuren 9 - 12 erläutert werden. In den Figuren 9 - 12 sind durch einzelne Kreise Messergebnisse dargestellt.

Die Erfindung wird anhand der folgenden Beispiele näher erläutert.

Die für die nachfolgend beschriebenen Versuche verwendeten Feldspatgranulate besitzen die folgende Zusammensetzung:

| **Oxide** | **Gew.-%** |
|---|---|
| SiO₂ | 56 - 64 |
| Al₂O₃ | 20 - 23 |
| Na₂O | 6 - 9 |
| K₂O | 6 - 8 |
| CaO | 0,3 - 0,6 |
| TiO₂ | 0,0 - 0,1 |

Mit Hilfe einer Grafik-Software, z.B. der Software Freeform, werden die Grenzflächen der Schmelz-Dentin-Grenzschichten der Oberkiefer- und Unterkieferzähne, jeweils vom rechtsseitigen Dreier bis zum linksseitigen Dreier, beispielsweise von Vitalife-Zähnen, sowohl der pyknischen, leptosomen und athletischen Form der Zähne, zusammengelegt und so die Grenzflächenform generiert. Alternativ können von natürlichen Zähnen die Grenzschichten durch Abtragung der Schmelzschicht herausgearbeitet werden und anschließend die Oberflächen mit einem handelsüblichen Scanner, z.B. mit einem Streifenlichtscanner, eingescannt werden und mit Hilfe der Software zusammengelegt werden. Unter Berücksichtigung des für das Granulat bei den später beschriebenen Press- und Sinterbedingungen bekannten Schrumpfungswert, der in zusätzlichen Versuchen bestimmt wird, wird die Bogenform mit Hilfe der Software vergrößert berechnet, damit nach der Fertigstellung des Blockes ein genaues Abbild der berechneten Grenzschicht vorliegt. Mittels der Software wird eine STL-Datei generiert. Die vergrößerte Bogenform wird mittels CAD/CAM-Verfahren unter Zuhilfenahme einer CNC-Fräsmaschine aus einem Messing-Block herauspräpariert, wobei an der Unterseite der Bogenform ein rechteckiges Prisma als Führung angebracht wird, das als Halterung und Führung des Pressstempels dient.

Weitere Möglichkeiten zur Herstellung eines Presstempels sind dem Fachmann bekannt.

Eine erste Möglichkeit der Blockherstellung ist die Folgende:
In einer Edelstahlform mit den dem Pressstempel angepassten Innenmaßen 16,6 mm x 21 mm, die Höhe betrug 63 mm und die Wanddicken ca. 10 mm,, wird granuliertes, mit einem handelsüblichen Binder versetzes Feldspatmaterial der transluzenten äußeren Schicht eingefüllt. Nachdem der Pressstempel eingeführt wurde, wird der Pressstempel in einer Presse mit einer Kraft von 1,5 kN belastet und das Granulat vorgepresst. Nach der Entlastung des Pressstempels und der Entnahme desselben wird das zweite Granulat, das sich vom ersten bereits verfestigten in der Helligkeit durch Zugabe entsprechender färbender Oxide unterscheidet und den Schmelz bildet, in die vergrößerte Bogenform im Grünling und ein wenig darüber hinaus gefüllt. Ein zweiter Presstempel mit einer ebenen Oberfläche wird eingeführt und mit 1,5 kN belastet, so dass das zweite Granulat äquivalent zum ersten Granulat verpresst wird. Anschließend wird der Pressstempel entfernt und der Grünling aus der Matrize herausgestoßen. Die Masse des Grünlings beträgt ca. 9,5 g.

Eine weitere Möglichkeit zur Herstellung eines Blockes wird im Folgenden beschrieben, wobei das verwendete Granulat identisch zur vorherigen Beschreibung ist. Der Pressstempel wird zuvor entsprechend den verwendeten Prozessparametern berechnet.
1.) Füllen der Pressform der Größe 21,0mm*16,5mm mit einer Menge von ca. 5,25g Material und
   Absenken des in der Pressform dagegenhaltenden Pressstempels (Unterstempel) auf die Füllmenge der transluzenten Farbkomponente (von der Sollhöhe des quaderförmigen Endproduktes und der Schüttdichte abhängig). Typischerweise 18-35mm.
2.) Verdichten der eingefüllten Masse mit einer zweiten Pressform, deren Oberfläche in 3 Raumrichtungen vergrößert angepasst gegenüber der des fertigen Blockes ist, so dass das verdichtete Material formstabil in der Pressform verbleibt. Typischerweise so, dass der Abstand der zweiten Pressform zum Unterstempel, zwischen 14 und 25mm beträgt. Dabei bestimmt die Menge des zu verpressenden Materials und der Abstand der Stempel die Dichte des vorverdichteten formstabilen Formkörpers.
3.) Auffüllen der vorverdichteten formstabilen Form mit dem dunkleren Material entsprechend der zu erreichenden Sollhöhe des Endproduktes nach 1.). Typischerweise 4,25g.
4.) Verdichten der Gesamteinheit auf eine spezifische Enddichte von typischerweise 1,54g/cm³ (hier 22,4 kN) auf eine Höhe von typischerweise 16,7mm, sodass der Endformkörper formstabil ist. Der Pressdruck /-kraft ist von den Material- und Prozessparametern abhängig.
5.) Entformen der Gesamteinheit aus der Pressform

Die so erhaltenen Blöcke, bestehend aus einer hellen, transluzenten Schicht außen und einer dunklen, opaken Schicht innen, werden entsprechend dem verwendeten Binder entbindert. Dieser Vorgang ist dem Fachmann bekannt und abhängig vom verwendeten Binder sowie der gewählten Korngröße des keramischen Materials. Nach dem Entbindern bei 700 °C bis 800 °C wird der nun erhaltene poröse Block unter Vakuum bei ca. 1200 °C dicht gesintert, so dass keine Poren und Hohlräume mehr vorhanden sind. Nach dem dicht-Sintern wird an den Block eine Halterung für eine Cerec-Schleifmaschine der Firma Sirona angebracht, damit der Block in der Maschine geschliffen werden kann. Durch eine entsprechende Software, die die Rotation als auch die translatorische Bewegung der Restauration im Block ermöglicht, kann eine frei wählbare Position einer Restauration im Block mit individuellem Grenzflächenverlauf von Schmelz und Dentin hergestellt werden.

## Patentansprüche

1. Formkörper aus formstabilisiertem Material, das mindestens einen ersten Bestandteil und einen zweiten Bestandteil (14) aufweist, wobei der zweite Bestandteil (14) eine andere Pigmentierung als der erste Bestandteil aufweist und der zweite Bestandteil (14) in dem ersten Bestandteil, unter Ausbildung einer Grenzfläche (16) so angeordnet ist, dass die Grenzfläche (16) eine räumlich gekrümmte Fläche darstellt,
wobei die Grenzfläche (16) im wesentlichen durch eine Parabelschar beschrieben ist, wodurch der zweite Bestandteil (14) in einer Querschnittsebene (36, 38, 40) eine im wesentlichen parabelförmige Grenzlinie (42, 44, 46) zum ersten Bestandteil aufweist,
die parabelförmige Grenzlinie (42, 44, 46) ein Maximum (48) aufweist, wobei die Maxima der parabelförmigen Grenzlinien (42), (44), (46) entlang einer Symmetrieebene (50) über eine Breite des Formkörpers (10) stetig abnehmen,
und bei welchem die Abnahme der Parabelmaxima (48) über mindestens 50%, vorzugsweise mindestens 75% des zweiten Bestandteils (14) in Verjüngungsrichtung (52) erfolgt.

2. Formkörper nach Anspruch 1, wobei der erste und zweite Bestandteil eine Keramik, insbesondere eine Feldspat- oder Oxidkeramik ist.

3. Formkörper nach Anspruch 2, wobei die Feldspatkeramik Metalloxide umfasst, die ausgewählt sind aus der Gruppe bestehend aus SiO₂, Al₂O₃, Na₂O, K₂O, gegebenenfalls ergänzt durch Zugabe von Pigmenten und anorganischen Füllstoffen oder
wobei die Oxidkeramik Metalloxide umfasst, die ausgewählt sind aus der Gruppe bestehend aus SiO₂, Al₂O₃, ZrO₂, stabilisiert durch verschiedene Verbindungen wie zum Beispiel Y₂O₃, CeO₂, gegebenenfalls ergänzt durch Zugabe von Pigmenten bzw. Verbindungen färbender Ionen.

4. Formkörper nach mindestens einem der Ansprüche 1 bis 3, wobei der Formkörper dichtgesintert oder porös gesintert worden ist.

5. Formkörper nach mindestens einem der Ansprüche 1 bis 4, wobei das sinterfähige Material vorzugsweise bei der Formgebung mit einem Binder versehen ist, insbesondere
ausgewählt aus der Gruppe bestehend aus Acrylat (-en), PVA, Cellulosederivate.

6. Formkörper nach Anspruch 1, wobei der erste und zweite Bestandteil ein thermoplastischer und/oder duroplastischer Kunststoff, insbesondere ein Acrylatpolymer ist.

7. Formkörper nach Anspruch 1, bei welchem der zweite Bestandteil (14) in zueinander parallelen Querschnittsebenen (36, 38, 40) jeweils eine parabelförmige Grenzlinie (42, 44, 46) aufweist, wobei die Grenzlinie (42, 46, 48) vorzugsweise in allen zueinander parallelen Querschnittsebenen (36, 38, 40) parabelförmig ist oder
bei welchem die Parabelmaxima (48) der Grenzlinien (42, 44, 46), ausgehend von einer Haupt-Querschnittsebene (22) in einer Verjüngungsrichtung (52), die vorzugsweise senkrecht zur Haupt-Querschnittsebene (22) ist, vorzugsweise stetig abnehmen oder
bei welchem die Parabelmaxima (48) in einer gemeinsamen in Verjüngungsrichtung (52) verlaufenden Ebene (50) liegen, bei der es sich insbesondere um eine Symmetrieebene (50) des zweiten Bestandteils (14) handelt.

8. Formkörper nach mindestens einem der Ansprüche 1 bis 7, bei welchem die parabelförmige Grenzlinie (42, 44, 46) an zumindest einem Ende (34) der beiden Parabeläste in eine Kurve entgegengesetzter Krümmung übergeht.

9. Verfahren zur Herstellung eines Formkörpers aus einem insbesondere sinterfähigen Material oder Kunststoffmaterial nach mindestens einem der Ansprüche 1 bis 8, der mindestens einen ersten und mindestens einen zweiten Bestandteil aufweist, wobei
a) der mindestens erste Bestandteil in eine Form eingefüllt wird,
b) in den eingefüllten mindestens ersten Bestandteil des insbesondere sinterfähigen Materials oder Kunststoffmaterials eine Mulde mit einer Oberfläche eingepresst und
c) die Oberfläche eine räumlich gekrümmte Grenzfläche mit dem
d) in die Mulde eingefüllten mindestens zweiten Bestandteils bildet
e) wobei sich gegebenenfalls an den Schritt c) ein Sinterschritt anschließt.

10. Verfahren nach Anspruch 9, wobei im Falle des Kunststoffmaterials der erste und zweite Bestandteil unter Temperatur und gegebenenfalls Druck ausgehärtet werden.

11. Verwendung des Formkörpers nach einem der Ansprüche 1 bis 8 zur Herstellung einer Dentalrestauration, wobei die Dentalrestauration gegebenenfalls mittels CAD/CAM-Methoden konstruiert und hergestellt wird.

## Claims

1. A molded member made of form-stabilized material comprising at least a first component and a second component (14), wherein said second component (14) has a different pigmentation from that of the first component, and the second component (14) is arranged within the first component to form an interface (16) in such a way that said interface (16) represents a surface curved in space, wherein the interface (16) is essentially described by a family of parabolas, whereby said second component (14) has an essentially parabolic border line (42, 44, 46) towards said first component in a cross-sectional plane (36, 38, 40), said parabolic border line (42, 44, 46) has a maximum (48), in which the maximums of the parabolic border lines (42), (44), (46) decrease continuously along a plane of symmetry (50) over a width of the molded member (10), and in which the decrease of the maximums of the parabola (48) goes through at least 50%, preferably through at least 75%, of the second component (14) in the taper direction (52).

2. The molded member according to claim 1, wherein the first and second component is a ceramic material, especially a feldspar or oxide ceramic material.

3. The molded member according to claim 2, wherein said feldspar ceramic material comprises metal oxides selected from the group consisting of SiO₂, Al₂O₃, Na₂O, K₂O, optionally supplemented by adding pigments and inorganic fillers, or
wherein said oxide ceramic material comprises metal oxides selected from the group consisting of SiO₂, Al₂O₃, ZrO₂ stabilized by various compounds, such as Y₂O₃, CeO₂, optionally supplemented by adding pigments or compounds of coloring ions.

4. The molded member according to at least one of claims 1 to 3, wherein said molded member has been dense-sintered, or porous-sintered.

5. The molded member according to at least one of claims 1 to 4, wherein said sinterable material is preferably provided with a binder during the shaping, especially one selected from the group consisting of acrylate(s), PVA, cellulose derivatives.

6. The molded member according to claim 1, wherein said first and second components are a thermoplastic and/or thermosetting material, especially an acrylate polymer.

7. The molded member according to claim 1, in which said second component (14) respectively has a parabolic border line (42, 44, 46) in parallel cross-sectional planes (36, 38, 40), preferably wherein said border line (42, 46, 48) is parabolic in all mutually parallel cross-sectional planes (36, 38, 40), or
wherein the maximums of the parabola (48) of said border lines (42, 44, 46) decrease, preferably continuously, from a main cross-sectional plane (22) in a taper direction (52), which is preferably perpendicular to the main cross-sectional plane (22), or
wherein the maximums of the parabola (48) lie in a common plane (50) extending in the taper direction (52), in particular which is a plane of symmetry (50) of the second component (14).

8. The molded member according to at least one of claims 1 to 7, wherein said parabolic border line (42, 44, 46) merges into a curve of opposite curvature on at least one end (34) of the two branches of the parabola.

9. A process for preparing a molded member consisting of in particular a sinterable material or plastic material according to at least one of claims 1 to 8, which has at least one first and at least one second component, wherein
a) said at least one first component is filled into a mold;
b) a depression having a surface is pressed into the filled-in at least one first component of said material, especially sinterable material or plastic material; and
c) said surface forms an interface curved in space towards
d) the at least one second component filled into the depression;
e) wherein step c) is followed, for sinterable matrerials, by a sintering step.

10. The process according to claim 9, wherein the first and second components are cured at an elevated temperature and optionally under pressure in the case of a plastic material.

11. Use of the molded member according to any of claims 1 to 8 for preparing a dental restoration, wherein said dental restoration is optionally constructed and prepared by means of CAD/CAM methods.

## Revendications

1. Pièce moulée en matériau stabilisé dimensionnellement ayant au moins un premier composant et un deuxième composant (14), dans laquelle ledit deuxième composant (14) présente une pigmentation différente de celle du premier composant, et ledit deuxième composant (14) est arrangé au sein dudit premier composant pour former une interface (16) de telle manière que l'interface (16) représente une surface incurvée spatialement,
dans laquelle ladite interface (16) est décrite sensiblement par une famille de paraboles, ledit deuxième composant (14) ainsi présentant une frontière sensiblement parabolique (42, 44, 46) avec le premier composant dans un plan transversal (36, 38, 40),
ladite frontière parabolique (42, 44, 46) présentant un maximum (48), où les maximums desdites frontières paraboliques (42), (44), (46) diminuent constamment le long d'un plan de symétrie (50) sur la largeur de la pièce moulée (10),
et dans laquelle ladite diminution des maximums des paraboles (48) a lieu sur au moins 50 %, de préférence au moins 75 %, du deuxième composant (14) dans la direction de rétrécissement (52).

2. Pièce moulée selon la revendication 1, dans laquelle lesdits premier et deuxième composants sont une céramique, notamment une céramique feldspathique ou oxydique.

3. Pièce moulée selon la revendication 2, dans laquelle ladite céramique feldspathique comprend des oxydes métalliques choisis dans le groupe consistant en SiO₂, Al₂O₃, Na₂O, K₂O, éventuellement supplémentés par l'addition de pigments et d'agents de charge inorganiques, ou
dans laquelle ladite céramique oxydique comprend des oxydes métalliques choisis dans le groupe consistant en SiO₂, Al₂O₃, ZrO₂ et stabilisés par des composés divers, par exemple Y₂O₃, CeO₂, éventuellement supplémentés par l'addition de pigments ou de composés d'ions colorants.

4. Pièce moulée selon au moins une des revendications 1 à 3, dans laquelle ladite pièce moulée provient d'un frittage à densité maximale ou d'un frittage poreux.

5. Pièce moulée selon au moins une des revendications 1 à 4, dans laquelle le matériau frittable est de préférence pourvu d'un liant pendant le moulage, notamment un liant choisi dans le groupe consistant en acrylate(s), PVA, dérivés de cellulose.

6. Pièce moulée selon la revendication 1, dans laquelle lesdits premier et deuxième composants sont une matière thermoplastique et/ou thermodurcissable, notamment un polymère d'acrylate.

7. Pièce moulée selon la revendication 1, dans laquelle ledit deuxième composant (14) présente une frontière parabolique (42, 44, 46) dans chacun de plusieurs plans transversaux mutuellement parallèles (36, 38, 40), dans laquelle ladite frontière (42, 44, 46) est de préférence parabolique dans chacun des plans transversaux mutuellement parallèles (36, 38, 40), ou
dans laquelle les maximums des paraboles (48) des frontières (42, 44, 46) diminuent, de préférence constamment, à partir d'un plan transversal principal (22) dans une direction de rétrécissement (52), qui est de préférence perpendiculaire au plan transversal principal (22), ou
dans laquelle les maximums des paraboles (48) sont situés dans un plan commun (50) orienté dans la direction de rétrécissement (52), qui est notamment un plan de symétrie (50) du deuxième composant (14).

8. Pièce moulée selon au moins une des revendications 1 à 7, dans laquelle la frontière parabolique (42, 44, 46) passe en une courbe de courbure opposée à au moins une extrémité (34) des deux branches de parabole.

9. Procédé pour fabriquer une pièce moulée en un matériau qui est notamment frittable ou en une matière plastique selon au moins une des revendications 1 à 8, qui présente au moins un premier composant et au moins un deuxième composant, dans lequel
a) ledit au moins un premier composant est chargé dans un moule,
b) un creux ayant une surface est pressé dans ledit au moins un premier composant chargé du matériau qui est notamment frittable ou de la matière plastique, et
c) la surface forme une interface incurvée spatialement avec ledit
d) au moins un deuxième composant chargé dans le creux,
e) l'étape c) étant suivie d'une étape de frittage pour les matériaux frittables.

10. Procédé selon la revendication 9, dans lequel lesdits premier et deuxième composants sont durcis au chaud et éventuellement sous pression dans le cas de la matière plastique.

11. Utilisation de ladite pièce moulée selon au moins une des revendications 1 à 8 pour fabriquer une restauration dentaire, où ladite restauration dentaire est éventuellement construite et fabriquée au moyen de méthodes CAD/CAM.
